## Europäisches Patentamt
## European Patent Office
## Office européen des brevets

(19)

(11) Veröffentlichungsnummer: **0 379 708 B1**

(12) **EUROPÄISCHE PATENTSCHRIFT**

(45) Veröffentlichungstag der Patentschrift: **07.12.94**

(21) Anmeldenummer: **89123535.0**

(22) Anmeldetag: **20.12.89**

(51) Int. Cl.[5]: **C12N 1/14**, C12P 21/04,
//(C12N1/14,C12R1:645),
(C12P21/04,C12R1:645)

(54) **Pilzart Tolypocladium varium und Verfahren zur Herstellung des Antibioticumkomplexes Cyclosporin und/oder seiner Bestandteile.**

(30) Priorität: **20.12.88 HU 649788**

(43) Veröffentlichungstag der Anmeldung:
**01.08.90 Patentblatt 90/31**

(45) Bekanntmachung des Hinweises auf die
Patenterteilung:
**07.12.94 Patentblatt 94/49**

(84) Benannte Vertragsstaaten:
**DE**

(56) Entgegenhaltungen:

**ANN. NEW YORK ACAD. SCIENCES (Biochem. Eng. 5), Band 506, 1987, Seiten 657-662; S.N. AGATHOS et al.: "The fungal production of cyclosporine"**

**"The Merck Index", Eleventh Edition, 1989, Seite 431, abstract no. 2759, Merck & CO., Inc., Rahway, N.J., US; "Cyclosporins"**

(73) Patentinhaber: **BIOGAL GYOGYSZERGYAR**
**Pallagi ut 13**

**H-4042 Debrecen (HU)**

(72) Erfinder: **Jekkel née Bokany, Antonia, Dr.**
**Damjanich u. 38**
**H 1071 Budapest (HU)**
Erfinder: **Ambrus, Gabor, Dr.**
**Csalan u. 45/b**
**H 1025 Budapest (HU)**
Erfinder: **Toth-Sarudy, Eva, Dr.**
**Pasareti ut 161**
**H 1026 Budapest (HU)**
Erfinder: **Szabo, Istvan Mihaly, Dr.**
**Szasz K. u. 2**
**H 1027 Budapest (HU)**
Erfinder: **Huelber, née Dobos, Agota**
**Boerzsoeny u. 4/II**
**H-1098 Budapest (HU)**
Erfinder: **Andor, Attila**
**Peres u. 4**
**H-1221 Budapest (HU)**
Erfinder: **Albrecht, Károly**
**Mexikoi ut 44**
**H-1145 Budapest (HU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 379 708 B1

Erfinder: **Koenczoel, Kálmán, Dr.**
**Dunyov u. 16**
**H-1134 Budapest (HU)**
Erfinder: **Szell, Valéria, Dr.**
**Balazs u. 24/b**
**H-1147 Budapest (HU)**
Erfinder: **Tomori, née Joszt, Eva, Dr.**
**Berda J. u. 44**
**H-1043 Budapest (HU)**
Erfinder: **Moravcsik, Imre**
**Mester u. 38**
**H-1095 Budapest (HU)**
Erfinder: **Polya, Kálmán, Dr.**
**Pechy u. 9**
**Debrecen (HU)**
Erfinder: **Erdei, János, Dr.**
**Andahazi u. 8**
**Debrecen (HU)**
Erfinder: **Kiss, Lajos**
**Micsurin u. 171**
**Debrecen (HU)**
Erfinder: **Nagy, Károly**
**Bethlen u. 48**
**Debrecen (HU)**
Erfinder: **Palotás, Béla**
**Dozsa Gy. u. 17**
**Debrecen (HU)**
Erfinder: **Deli, née Konszky, Etelka, Dr.**
**Jeriko u. 8**
**Debrecen (HU)**
Erfinder: **Buzási, Károly**
**Kishegessy u. 46**
**Debrecen (HU)**
Erfinder: **Molnár, née Antal, Anik , Dr.**
**Illyes Gy. u. 20**
**Debrecen (HU)**
Erfinder: **Sántha, György**
**Istvan u. 61**
**Debrecen (HU)**
Erfinder: **Szászhegyesi, Vilma**
**Hatvani I. u. 28**
**Debrecen (HU)**

(74) Vertreter: **Beszédes, Stephan G., Dr.**
**Patentanwalt**
**Postfach 11 68**
**D-85201 Dachau (DE)**

**Beschreibung**

Die Erfindung betrifft einen neuen Mikroorganismenstamm Tolypocladium varium und ein Verfahren zur Herstellung des Antibioticumkomplexes Cyclosporin und seiner Bestandteile Cyclosporin A, Cyclosporin B und Cyclosporin C durch durch aerobes Gären erfolgendes Züchten der genannten Pilzart.

Die einzelnen Komponenten dieses Antibioticumkomplexes sind aus 11 Aminosäuren bestehende cyclische, neutrale, apolare Oligopeptide, die sich nur durch 1 oder 2 der am Aufbau teilnehmenden Aminosäuren voneinander unterscheiden.

Von den Bestandteilen des Antibioticumkomplexes wurden das Cyclosporin A und das Cyclosporin C von A. Rüegger und Mitarbeitern (Helv. Chim. Acta 59 [1976], 1075) und die Cyclosporine B, D, E und G von R. Traber und Mitarbeitern (Helv. Chim. Acta 60 [1977], 1 568; belgische Patentschrift 879 402) aus der Kultur eines von ihnen früher als Trichoderma polysporum (Link ex Pers.) Rifai identifizierten Pilzes (NRRL 8044) zuerst isoliert. Später wurde die systematische Einordnung des betreffenden Pilzstammes (NRRL 8044) präzisiert und seitdem wird er im Schrifttum als Tolypocladium inflatum Gams bezeichnet.

Zur Zeit sind bereits 25 Cyclosporine bekannt, die mit den Buchstaben von A bis Z bezeichnet werden (Helv. Chim. Acta 70 [1987], 13).

Von den Bestandteilen ist, was die Wirkung betrifft, das selektiv immunsuppressiv wirksame Cyclosporin A am wertvollsten.

Das Cyclosporin A wurde zuerst als schwach antimykotisch wirkendes Antibioticum bekannt, seine bedeutende immunsuppressive Wirkung wurde erst später erkannt (J. F. Borel und Mitarbeiter: Immunology 32 [1977], 1 017).

Mit ganzen Reihen von Versuchen in vivo und in vitro konnte nachgewiesen werden, daß die Cyclosporine A, C und G spezifischere Wirkstoffe sind als die bisher bekannten immunsuppressiven Arzneimittel. Es erwies sich, daß das Cyclosporin A sowohl die humorale als auch die zelluläre Immunantwort hemmt. Bei der Untersuchung seines Wirkungsmechanismus wurde festgestellt, daß es die Proliferation der T-Zellen hemmt und die Synthese des Interleukin-2 unterbricht.

Über die Verwendung des Cyclosporin A als Arzneimittel bei Organverpflanzungen am Menschen wurde 1978 berichtet. Die erste Anwendung wurde im Zusammenhang mit einer Nierenverpflanzung (R. J. Calne und Mitarbeiter: Lancet 1978/2, 1 323) und einer Knochenmarkübertragung (R. L. Powles und Mitarbeiter: Lancet 1978/2, 1 327) beschrieben. Durch die Verwendung von Cyclosporin A kann bei Organverpflanzungen das Abstoßen des Organs (Niere, Bauchspeicheldrüse, Leber, Herz, Lunge), bei Knochenmarkübertragungen das Abstoßen des Knochenmarks verhindert werden.

Auch wurde das Cyclosporin A erfolgreich zur Behandlung bestimmter Autoimmunkrankheiten (zum Beispiel Uveitis, rheumatoide Arthritis, Psoriase und Myasthenia gravis) verwendet.

Gemäß dem Patentschrifttum wurden zur Herstellung von Cyclosporin-Komplex enthaltenden Gärbrühen bisher Kulturen der folgenden Mikroorganismenstämme verwendet: Cylindocarpon lucidum Booth, NRRL 5 760(schweizerische Patentschrift 589 716); Tolypocladium inflatum Gams, NRRL 8 044 [nach älterer Nomenklatur; Trichoderma polysporum (Link ex Pers.) Rifai, schweizerische Patentschrift 603 790 und "THE MERCK INDEX" ELEVENTH EDITION, 1989, Seite 431, Abstract No. 2759, nach Bissett (1983) ist Tolypocladium inflatum W. Gams, 1971 synonym mit Tolypocladium niveum (Rostrup) Bissett comb. nov.] und Fusarium solani, MCI-1 549, MIC-1 550 (bekanntgemachte japanische Patentanmeldung 82 63093) und Tolypocladium inflatum ATCC 34 921 (ANN NEW YORK ACAD. OF SCIENCES (Biochem. Eng. 5), Band 506, 1987, Seiten 657 bis 662). Eine technische beziehungsweise industrielle Nutzung dieser Mikroorganismenstämme in Gärverfahren hat jedoch den Nachteil, daß die Gärungszeiten lang sind (11 bis 14 Tage), und der Wirkungsgrad der Isolierung des Komplexes aus der Gärbrühe verhältnismäßig niedrig ist (etwa 40%). Auch wird mit Ausnahme der letztgenannten Druckschrift wenig Cyclosporinkomplex erzeugt (etwa 20 bis 30 mg/ml), wobei auch in der letztgenannten Druckschrift mit dem Elternstamm von Tolypocladium inflatum nur etwa 150 mg/ml Cyclosporin-Komplett erzeugt wurden und nur mit der Mutante M6 die höchste Menge von 537 mg/ml zu erzielen war. Das letztere jedoch ausweislich der Fig. 2 auf Seite 661 auch nur unter Laboratoriumsbedingungen nach einer 16-tägigen Gärung.

Keine der obigen Druckschriften betrifft die Pilzart Tolypocladium varium und deren Verwendung.

Der Erfindung liegt die Aufgabe zugrunde, unter Behebung der Nachteile des Standes der Technik eine neue Pilzart und ein neues Verfahren zur Herstellung des Antibioticumkomplexes Cyclosporin und/oder seiner Bestandteile, durch welche der Antibioticum-Komplex Cyclosporin mit kürzerer Gärungszeiten und in größerer Menge erzeugt und aus der Gärbrühe mit höherem Wirkungsgrad isoliert werden kann, zu schaffen.

Das Obige wurde überraschenderweise durch die Erfindung erreicht.

3

Im Zuge eigener Forschungen wurde nach Mikroorganismenstämmen, mit denen der Cyclosporin-Antibioticumkomplex oder seine Bestandteile in hoher Konzentration und unter vorteilhafteren Bedingungen als bisher hergestellt werden kann, gesucht. Im Laufe von Reihenuntersuchungen, die sich auf viele tausend Pilzstämme erstreckten, konnte ein der Pilzart Tolypocladium angehörender Mikroorganismus, der in kürzerer Zeit und in größerer Menge den Antibioticum-Komplex Cyclosporin biosynthetisiert als die bekannten Stämme und der, wie taxonomische Untersuchungen zeigen, mit keinem der bekannten Cyclosporin erzeugenden Pilzstämme identisch ist, ausgewählt werden. Der neue Stamm (CY/93), der eigenen Untersuchungen gemäß als Mitglied eines neuen unterscheidbaren Taxons des Genus Tolypocladium anzusprechen ist, wurde unter der Bezeichung Tolypocladium varium species nova als holotyper Stamm beschrieben.

Die Erfindung beruht demnach auf der überraschenden Feststellung, daß durch Gären des bisher nicht bekannten Typenstammes Tolypocladium varium species nova (Hausbezeichnung: CY/93), der am 15. Mai 1987 unter der Nummer NCAIM(P)F 001005 in der Nationalen Sammlung Landwirtschaftlicher und Industrieller Mikroorganismen der Budapester Universität für Gartenbau und Lebensmittelindustrie hinterlegt worden ist, eine Gärbrühe mit sehr hoher Cyclosporinkonzentration gewonnen werden kann. Die Gärbrühe enthält neben dem Hauptprodukt Cyclosporin A in geringen Mengen Cyclosporin B und Cyclosporin C.

Gegenstand der Erfindung ist daher der Mikroorganismenstamm Tolypocladium varium sp. nov. CY/93, hinterlegt am 15. Mai 1987 unter der Nummer NCAIM(P)F 001005 in der Nationalen Sammlung Landwirtschaftlicher und Industrieller Mikroorganismen der Budapester Universität für Gartenbau und Lebensmittelindustrie.

Die zur Systematisierung heranziehbaren Merkmale des isolierten neuen Pilzstammes werden im folgenden mit den wichtigsten der diagnostischen Charakteristika der bekannten Tolypocladiumarten verglichen.

Das Genus Tolypocladium wurde 1971 von Gams als ein neues Genus der bodenbewohnenden Moniliales beschrieben. Seine charakteristischen Eigenschaften sind: Langsames Wachstum, polsterförmige weiße oder weißliche Kolonien, terminale und laterale, zum Teil an kurzen Seitenzweigen angeordnete Phialiden mit stark gequollenem Grund, an dem ein fadenartiger, häufig bogenförmig geneigter Hals wahrnehmbar ist, der in einer kleinen, einzelligen Konidie (Köpfchen) endet. Innerhalb dieses Genus wurden von Gams die folgenden 3 neuen Arten unterschieden: T. geodes, T. cylindrosporum und T. inflatum. T. geodes weist einen auffallenden, an Actinomyceten erinnernden Erdgeruch auf, der dem erfindungsgemäßen T. varium sp. nov. CY/93 völlig fehlt. Außerdem sind die Trägerzellen von T. geodes wesentlich schmaler als die des erfindungsgemäßen T. varium sp. nov. CY/93. Die Konidien von T. cylindrosporum sind charakteristisch lang und gestreckt, die Konidien des erfindungsgemäßen T. varium sp. nov. CY/93 dagegen sphärisch oder schwach gestreckt. Die Konidien von T. inflatum sind elliptisch und länger als die des erfindungsgemäßen T. varium sp. nov. CY/93. Die Phialiden von T. inflatum sind entweder unmittelbar and der Hyphe oder an 2 bis 3 $\mu$m langen Halterzellen charakteristisch quirlständig angeordnet. Für die Phialiden des erfindungsgemäßen T. varium sp. nov. CY/93 ist die Quirlständigkeit nicht charakteristisch und wird nur vereinzelt beobachtet. Die Kolonien aller bisher bekannten Tolypocladiumarten sind weiß, was in erster Linie auf das weiße, watteartige Luftmyzel zurückzuführen ist. Die Unterseite der Kolonien ist gelblichgrau, farblos oder gelb, ein charakteristisches lösliches Pigment wird nicht erzeugt. Auch dieser Hinsicht weicht das erfindungsgemäße T. varium sp. nov. CY/93 von den bekannten Arten ab: auf Glucose- und Peptonnährböden sowie auf Malzextrakt/Hefeextrakt-Nährböden erzeugt der Pilz mit temporaler Variabilität ein dunkles, tiefgraues beziehungsweise schwarzes Pigment.

Beim systematischen Vergleich des neuen Stammes mit den bekannten Tolypocladiumarten wurde vor allem das folgende Schrifttum herangezogen: Gams, W.: Persoonia 6 [1971], 185 bis 191; Barron, G. L.: Can. J. Bot. 5R [1980], 439 bis 442 und Bissett, J.: Can. J. Bot. 61 [1982], 1 311 bis 1329.

Die diagnostischen Merkmale des erfindungsgemäßen Tolypocladium varium sp. nov. CY/93 können wie folgt zusammengefaßt werden. Die 10 Tage alten Kolonien haben einen Durchmesser von 10 bis 25 mm, ihre Oberfläche ist von einem watteartig weißen, reich sporulierenden Luftmyzel bedeckt. Die Unterseite der Kolonien ist gelblichgrau oder schmutziggrau. Auf manchen komplexen Nährböden wird ein dunkelgraues lösliches Pigment gebildet. An den Hyphen des Luftmyzels verläuft die Sporenbildung sowohl terminal als auch lateral. Die Phialiden sind vereinzelt oder manchmal auch quirlständig an den Hyphen oder an kurzen (2 bis 3 $\mu$m langen) Halterzellen angeordnet. Die Phialiden bestehen aus einem gequollenen Basisteil (2 bis 4 x 2 bis 3 $\mu$m) und einem langen, fadenartigen Hals (2 bis 4 x 0,5 bis 0,6 $\mu$m). Die Konidien der Köpfchen sind klein (2 bis 3 x 1,3 bis 2,2 $\mu$m), im allgemeinen sphärisch und glatt. Das Species-Attribut "varium" bezieht sich auf die Variabilität der Phialiden. Der erfindungsgemäße Stamm Tolypocladium varium sp. nov. CY/93 zeigt in seinem Lebenszyklus eine bestimmte Ähnlichkeit mit dem Genus Harposporium. Er verwertet Raffinose nicht, wächst jedoch gut in Gegenwart der Kohlenstoffquellen Mannit, Inosit, Saccharose, Fructose, Rhamnose, Galactose, Glucose, Arabinose und Xylose. Auf Nähr- und Malzagar entwickeln sich seine Kulturen schwach, auf Soja-, Czapek- und Kartoffelagar wachsen sie kräftig. Hafer-Hefeextrakt, Glucose-Hefeextrakt und Haferagar sind zur Aufrechterhaltung nicht allzusehr geeignet. Tolypocladium varium sp.nov. CY/93 weicht von Tolypocladium trigonosporum scharf ab, da das letztere trigonale Konidien, die schwach konkave Ränder aufweisen, erzeugt und es weicht auch von Tolypocladium balanoides ab, da das letztere lageninforme seitliche Phialiden erzeugt.

Darüberhinaus unterscheidet sich der Holotypstamm (CY/93) des Tolypocladium varium n. sp. vom authentischen Typstamm (CBS 714.70) von T. inflatum auch darin, daß der letztere auf Malz-Extrakt-Agar ein reichliches weißes Luftmyzel bildet, wohingegen der Stamm T. varium sp. nov. CY/93 kein Luftmyzel entwickelt. Der Typstamm T. inflatum erzeugt ein rötlichbraunes Endopigment in seinem Substratmyzel auf Eisen-Pepton-Agar. Demgegenüber bleibt das Substratmyzel von T. varium sp. nov. CY/93 blaßgelb. Der Stamm T. varium sp. nov. CY/93 verwertet die Arabinose nicht, wohingegen der Stamm T. inflatum die Arabinose verwertet. Der Stamm T. inflatum entwickelt mit Natriumnitrit ein reichliches Myzel, wohingegen der Stamm T. varium sp. nov. CY/93 kaum wächst.

Ein Vergleich mit den authentischen Stämmen von anderen Tolypocladiumarten sowie mit den Stämmen Trichoderma polysporum ATCC 16641 und Cylindrocarpon lucidum NRRL 5760 und damit auch eine scharfe Abtrennbarkeit sind in der folgenden Tabelle dargestellt.

## Tabelle

| | Tolypocladium varium CY/93 | Tolypocladium cylindrosporum CBS 717.70 | Tolypocladium cylindrosporum CBS 718.70 | Tolypocladium geodes CBS 721.70 | Tolypocladium geodes 722.70 | Trichoderma polysporum ATCC 16641 | Cylindrocarpon lucidum NRRL 5760 |
|---|---|---|---|---|---|---|---|
| Cellulose-Zersetzung | - | - | - | - | - | +++ | +++ |
| Luftmyzel: grün oder blaulichgrün | - | - | - | - | | ++- | +++ |
| Rotes lösliches Pigment auf King-Agar | - | - | - | - | - | - | +++ |
| Braunes Pigment auf Eisen-Pepton-Agar | - | - | - | - | - | - | +++ |
| Dunkelbraunes Pigment auf synthetischem Glycerin-Agar | - | - | - | - | - | +++ | - |
| Saccharose-Verwertung | +++ | +++ | +++ | +++ | +-+ | - | +++ |
| Inulin-Verwertung | - | - | - | - | - | - | +++ |
| Wachstum bei 5 $^{\circ}$C | +++ | +++ | ++- | +++ | +-+ | - | - |
| Wachstum bei 37 $^{\circ}$C | - | - | - | - | - | + | +++ |
| Resistenz gegenüber 60-minutiger Wärmebehandlung bei 50 $^{\circ}$C | +++ | -- | - | - | - | +++ | +++ |
| Säureerzeugung aus Glucose | +++ | +++ | $\pm$ | $\pm$ | +-+ | +++ | +++ |
| Natriumsalicylat-Verwertung | - | - | - | - | - | +++ | +++ |
| Natriummalonat-Verwertung | ++ | ++ | ++ | - | - | ++ | ++ |
| Natriumtartrat-Verwertung | + | ++ | ++ | - | - | ++ | ++ |
| Lila lösliches Pigment auf Tyrosin- und Leucin-Agar | - | - | - | +++ | +++ | - | - |

Gegenstand der Erfindung ist auch ein Verfahren zur Herstellung des Antibioticumkomplexes Cyclosporin und/oder seiner Bestandteile Cyclosporin A, Cyclosporin B und/oder Cyclosporin C durch Züchten eines die genannten Antibiotica biosynthetisierenden Pilzstammes in einem assimilierbare Kohlenstoff- und organische und anorganische Stickstoffquellen sowie Mineralsalze enthaltenden Nährmedium unter aeroben Bedingungen und Abtrennen der gebildeden Produkte, welches dadurch gekennzeichnet ist, daß als die genannten Antibiotica biosynthetisierender Pilzstamm der Mikroorganismenstamm Tolypocladium varium sp. nov. CY/93, hinterlegt am 15. Mai 1987 unter der Nummer NCAIM(P)F 001005 in der Nationalen Sammlung Landwirtschaftlicher und Industrieller Mikroorganismen der Budapester Universität für Gartenbau und Lebensmittelindustrie, im Nährmedium bei 25 bis 30°C gezüchtet wird sowie gegebenenfalls der erhaltene Antibioticumkomplex oder seine Bestandteile aus der Gärbrühe isoliert und gegebenenfalls gereinigt wird beziehungsweise werden.

Dieser Stamm ist infolge seines schnellen Wachstums besonders vorteilhaft. Saccharose, Glucose, Sorbose, Maltose, Fructose, Stärke, Glycerin sowie verschiedene Fette und Öle verwertet er als Kohlenstoffquelle gut. Als Stickstoffquellen kommen verschiedene organische und anorganische Stickstoffquellen, zum Beispiel Maisquellwasser, Peptone, Hefeextrakte, Fleischextrakte, Natriumnitrat, Ammoniumnitrat, Ammoniumsulfat und/oder verschiedene Aminosäuren, in Frage. Als Mineralsalze kommen zum Beispiel Kaliumchlorid, Magnesiumsulfat und/oder Kaliumdihydrogenphosphat in Betracht. Ferner kann das Nährmedium Spurenelemente, zum Beispiel Kupfer, Mangan und/oder Eisen, sowie Vitamine und schaumhemmende Stoffe enthalten.

Nach einer bevorzugten Ausführungsform des erfindungsgemäßen Verfahrens wird von dem erfindungsgemäßen Stamm Tolypocladium varium sp. nov. CY/93 eine Schrägagarkultur angelegt, mit deren Sporen- und Myzelsuspension ein Inoculum-Nährmedium beimpft, dann die 3 Tage alte Inoculum-zu der etwa 10-fachen menge Produktinsmedium zugegeben und das Produktionsmedium bei 25 bis 30°C, vorzugsweise 25°C, 5 bis 7 Tage lang bebrütet. Während der Gärung wird der pH-Wert auf 6,0 bis 2,5, vorzugsweise auf 5,2, gehalten. Die Gärung wird unter aeroben Bedingungen und kräftigem Rühren (950 bis 1 000 min$^{-1}$) durchgeführt. Der Luftbedarf der Kultur beträgt etwa 300 l/h.

Während der Gärung wird der Wirkstoffgehalt der Gärbrühe durch mikrobiologische Wertmessung und durch Hochdruckflüssigkeitschromatographie (HPLC) überwacht. Wenn der Wirkstoffgehalt sein Maximum erreicht hat, wird die Gärung abgebrochen und das Antibioticum durch Extraktions- und/oder Adsorptionsverfahrensweisen isoliert.

Zur mikrobiologischen Wertmessung wird die von Cyclosporin A auf manche Fadenpilze ausgeübte selektive Wachstumshemmung genutzt. Als Testorganismen sind Aspergillus niger, Aspergillus japanicus und Curvularia lunata am geeignetsten (M. Dreyfuss und Mitarbeiter: European J. Appl. Microbiol. 3 [1976] 125 bis 133). Zur Hochdruckflüssigkeitschromatographie-Analyse der Gärbrühe wird das Filtrag von im Volumverhältnis von 1 : 10 mit Methanol verdünnten Proben verwendet [Apparat: LKB, Pumpenmodell 2 150, UV-Detektor Modell 2 151; Messung bei 220 nm; Säule BST Si-100-C$_8$, 7 μm Säulenhöhe 25 cm, Innendurchmesser 0,4 cm; Elutionsflüssigkeit: Acetonitril und Phosphorsäuretriäthylamin (pH-Wert = 6,8) im Volumverhältnis von 65 : 35].

Die experimentellen Erfahrungen zeigten, daß der erfindungsgemäße Stamm Tolypocladium varium sp. nov. CY/93 den als Hauptprodukt Cyclosporin A und daneben als Nebenbestandteil Cyclosporin B und Cyclosporin C enthaltenden Antibioticumkomplex in sehr guter Ausbeute (950 μg/ml) erzeugt.

Die Abtrennung des Cyclosporinkomplexes aus der Gärbrühe wird zweckmäßig durch Extrahieren durchgeführt. Es ist vorteilhaft, vor der Extraktion das Myzel durch Filtrieren oder Zentrifugieren aus dem Nährmedium zu entfernen. Von der abgetrennten Biomasse wird das anhaftende Antibioticum zweckmäßig mit niederen Alkoholen, insbesondere Methanol und/oder mit Ketonen, vorzugsweise Aceton, abgewaschen, während zum Extrahieren der im Nährmedium gelösten und in der Waschflüssigkeit befindlichen Cyclosporine zweckmäßig mit Wasser nicht mischbare Lösungsmittel, zum Beispiel Äthylacetat, n-Butylacetat, Isobutylacetat, Dichlormethan, 1,2-Dichloräthan und/oder Chloroform, vorzugsweise n-Butylacetat, verwendet werden. Das durch Extraktion erhaltene Rohprodukt ist durch von dem Pilz produzierte rote und lila Pigmente verunreinigt. Diese können durch Adsorption an Aktivkohle oder Silicagel entfernt werden. In der Gärbrühe etwaig vorliegende Verunreinigungen mit Lipidcharakter, zum Beispiel Antischaummittel, können durch Verteilung des Rohproduktes zwischen Petroläther und 10 Gew.-% Wasser enthaltendem Methanol entfernt werden. Die lipidartigen Verunreinigungen gelangen in die Petrolätherphase. Aus der wäßrig-methanolischen Phase wird das Methanol durch Eindampfen unter Vakuum entfernt, die zurückbleibende wäßrige Phase wird mit Dichlormethan extrahiert und der Auszug eingedampft. Aus dem auf diese Weise gewonnenen gereinigten Produkt können das Cyclosporin A und in geringerer Menge gebildete weitere Cyclosporinbestandteile säulenchromatographisch oder durch Umkristallisieren abgetrennt werden. Bei der ersteren Alternative wird vorzugsweise zum Eluieren ein Chloroform-Methanol-Gemisch mit einem Gradienten mit steigendem Methanolgehalt oder Hexan-Aceton-Gemisch mit einem Gradienten mit steigendem Acetongehalt verwendet.

Im Vergleich zu den bekannten Verfahren weist das erfindungsgemäße Verfahren eine Reihe von Vorteilen auf, die zusammengefaßt wie folgt sind:

a) Der Hauptvorteil des erfindungsgemäßen Verfahrens besteht darin, daß der erfindungsgemäße Stamm Tolypocladium varium sp. nov. CY/93 ein Mehrfaches der mit den bekannten Verfahren herstellbaren Cyclosporinkomplexmenge erzeugt, unter Laboratoriumsbedingungen werden Mengen bis zu 950 $\mu$g/ml erreicht.

b) Der erfindungsgemäße Stamm Tolypocladium varium sp. nov. CY/93 erzeugt den Cyclosporinkomplex in einer günstigen Zusammensetzung (etwa 85 Gew.-% des Komplexes bestehen aus Cyclosporin A und deswegen kann die Gärbrühe einfacher und wirtschaftlicher aufgearbeitet werden, als dies bei den bekannten Verfahren der Fall ist.

Die isolierten Produkte können durch UV-, IR-, $^1$H-NMR- und $^{13}$C-NMR-spektroskopische Untersuchungen, durch Aminosäurenanalyse und durch Hochdruckflüssigkeitschromatographie (HPLC) identifiziert werden.

Die Erfindung wird an Hand der folgenden Beispiele näher erläutert.

Beispiel 1

Von einer auf Malzextrakt und Hefeextrakt enthaltendem Schrägagar 5-7 Tage lang gezüchteten Kultur des Stammes Tolypocladium varium sp. nov. CY/93 (NCAIM(P)F 001005) wird mit 5 ml 0,9 gew.-%-iger Kochsalzlösung eine Sporensuspension bereitet. Mit 1 ml dieser Sporensuspension werden 100 ml Inoculum-Nährmedium IC, die sich in einem Erlenmeyer-Kolben von 500 ml Volumen befinden, beimpft.

| Zusammensetzung des Inoculum-Nährmediums IC | |
|---|---|
| Glucose | 40 g |
| Kaseinpepton | 5 g |
| Natriumnitrat | 3 g |
| Kaliumdihydrogenphosphat | 2 g |
| Kaliumchlorid | 0,5 g |
| Magnesiumsulfat .7H$_2$O | 0,5 g |
| Eisen(II)sulfat .7H$_2$O in 1000 ml Leitungswasser | 0,01 g |

Das Nährmedium wird auf pH 5,2 eingestellt und danach bei 121 °C 25 Minuten lang sterilisiert.

Die Kultur wird auf einem Schütteltisch (340 min$^{-1}$, Auslenkung 3,5 cm) bei 25 °C 3 Tage lang geschüttelt. Mit je 5 ml der erhaltenen Inoculumkultur werden 15 Erlenmeyer-Kolben von 500 ml Volumen, in denen sich je 100 ml steriles Nährmedium FC$_1$ befinden, beimpft.

| Zusammensetzung des Nährmediums FC$_1$ | |
|---|---|
| Glucose | 80 g |
| Trypkasin | 40 g |
| Harnstoff | 2 g |
| Ammoniumsulfat | 12 g |
| Natriumnitrat | 3 g |
| Kaliumdihydrogenphosphat | 2 g |
| Kaliumchlorid | 0,5 g |
| Magnesiumsulfat .7H$_2$O | 0,5 g |
| Eisen(II)sulfat .7H$_2$O in 1000 ml Leitungswasser. | 0,01 g |

Das Nährmedium wird auf pH 5.2 eingestellt und danach bei 121 °C 25 Minuten lang sterilisiert.

Die Kolben werden auf einem Schütteltisch (340 min$^{-1}$, Auslenkung 3,5 cm) bei 25 °C geschüttelt. Die Änderung des Antibioticumgehaltes der Gärbrühe wird mit der mikrobiologischen Methode der Agardiffusion verfolgt.

Die Agardiffusionsmethode zur mikrobiologischen Wertmessung beruht auf der antimykotischen Wirkung von Cyclosporin A. Als Testorganismus wird Aspergillus niger verwendet. Die Aktivität des gebildeten Cyclosporinkomplexes wird gegen einen Cyclosporin-A-Standard gemessen. Die 2-8 $\mu$g/ml Cyclosporin A enthaltenden, mit wässrigem Alkohol bereiteten Standardlösungen ergeben auf dem Hefeextrakt und Glucose enthaltenden Agarmeßmedium Hemmzonen von 16-25 mm Durchmesser.

Die Gärung wird 168 Stunden lang fortgesetzt, dann wird der gebildete Antibiotikumkomplex aus der Gärbrühe abgetrennt.

Aus 1 Liter Gärbrühe, die bei Abbruch der Gärung 400 $\mu$g/ml Cyclosporinkomplex enthielt, werden die Antibiotica wie folgt gewonnen.

Die Zellen werden durch Filtrieren aus der Gärbrühe entfernt und mit 2x200 ml Methanol die anhaftenden Antibiotica abgewaschen. Aus der wässrig-methanolischen Waschflüssigkeit wird das Methanol unter vermindertem Druck entfernt und das erhaltene wässrige Konzentrat mit 2x50 ml n-Butylacetat extrahiert. Aus dem Filtrat der Gärbrühe wird der Cyclosporinkomplex mit 200 ml n-Butylacetat extrahiert. Die Extrakte werden vereinigt, über wasserfreiem Natriumsulfat getrocknet und unter vermindertem Druck bei 40 °C eingedampft. Das erhaltene Rohprodukt (3,7 g) wird in 10 ml Methanol gelöst, und die lipophilen Verunreinigungen werden an einem Gelbett aus Sephadex® LH-20 (Höhe 40 cm, Durchmesser 2,5 cm) durch Eluieren mit Methanol entfernt. Die den Cyclosporinkomplex enthaltenden Fraktionen werden unter vermindertem Druck bei 40 °C eingedampft. Der erhaltene Cyclosporinkomplex wird an einer aus 20 g Silicagel (Kieselgel 40, Reanal, Budapest) bereiteten Säule durch Eluieren mit einem Chloroform-Methanol-Gradienten steigenden Methanolgehaltes in seine Komponenten aufgetrennt. Die Elution wird mit 100 ml Chloroform begonnen und mit 100 ml-Mengen Elutionsmittel, dessen Methanolgehalt bei jedem Schritt um 0,5 Vol.-% ansteigt, fortgesetzt. Der Cyclosporingehalt der chromatographischen Fraktionen wird dünnschichtchromatographisch verfolgt (Kieselgelplatten 60 $F_{254}$, DC Alufolie, Merck; Entwickler: Äthylacetat und Isopropanol im Volumverhältnis von 95:5 Anfärbung: Joddampf). Das Cyclosporin A wird mit 2,0 Vol.-% Methanol enthaltenden Chloroform von der Säule abgelöst, Cyclosporin B folgt bei 2,5Vol.-% Methanolgehalt, und Cyclosporin C ist in der Fraktion mit 3,0 Vol.-% Methanol zu finden. Die die einzelnen Cyclosporinkomponenten in reiner Form enthaltenden chromatographischen Fraktionen werden im Vakuum eingedampft. Auf diese Weise erhält man 225 mg Cyclosporin A (Schmp.: 137-140 °C; $[\alpha]_D$: -189° /Methanol/); 25 mg Cyclosporin B (Schmp.: 149-151 °C) und 52 mg Cyclosporin C (Schmp.: 150-152 °C.

### Beispiel 2

Auf die im Beispiel 1 beschriebene Weise wird auf dem Schütteltisch eine Inoculumkultur bereitet. Mit 200 ml dieser Kultur werden 5 Liter Inoculum Nährmedium IC, die sich in einem Laborfermentor von 10 Liter Volumen befinden und bei 121 °C 45 Minuten lang sterilisiert wurden, beimpft. Die Kultur wird bei 25 °C mit einer Drehzahl von 750 min$^{-1}$ gerührt und dabei mit einer Luftmenge von 300 l/h belüftet.

Nach 72 Stunden langer Gärung werden 500 ml der erhaltenen Inoculumkultur zur Beimpfung von 5 Liter Nährmedium $FC_2$ verwendet, das sich in einem Laborfermentor von 10 l Volumen befindet und bei 121 °C 45 Minuten lang sterilisiert wurde.

| Zusammensetzung des Nährmediums $FC_2$ | |
| --- | --- |
| Glucose | 80 g |
| Trypkasin | 40 g |
| Harnstoff | 2 g |
| Ammoniumsulfat | 12 g |
| Natriumnitrat | 3 g |
| Kaliumdihydrogenphosphat | 2 g |
| Kaliumchlorid | 0,5 g |
| Magnesiumsulfat .7$H_2$O | 0,5 g |
| Kupfersulfat .7$H_2$O | 0,01 g |
| Mangan(II)sulfat . 7$H_2$O | 0,01 g |
| Eisen(II)sulfat .7$H_2$O in 1000 ml Leitungswasser. | 0,01 g |

Der pH-Wert des Nährmediums wird vor der Sterilisierung auf 5,2 eingestellt. Die beimpfte Kultur wird bei 25 °C mit einer Drehzahl von 750 min$^{-1}$ gerührt und dabei mit einer Luftmenge von 300 l/h belüftet. Die Gärung wird 144-168 Stunden lang fortgesetzt und nach Erreichen der maximalen Cyclosporinkonzentration abgebrochen.

Aus 4,8 Liter Gärbrühe, die am Ende der Gärung 500 $\mu$g/ml Cyclosporinkomplex enthielt, wird das Cyclosporin A wie folgt isoliert.

Die Zellen werden durch Filtrieren aus der Gärbrühe entfernt, der an den Zellen haftende Antibioticumgehalt wird mit 2x1 Liter Methanol ausgewaschen. Aus der Waschflüssigkeit wird das Methanol unter vermindertem Druck entfernt und der Wirkstoff aus dem wässrigen Konzentrat mit 2x250 ml n-Butylacetat extrahiert.

Aus dem Filtrat der Gärbrühe wird der Cyclosporinkomplex mit 2x500 ml n-Butylacetat extrahiert. Die Extrakte werden vereinigt, über wasserfreiem Natriumsulfat getrocknet und unter vermindertem Druck bei 40 °C eingedampft. Das erhaltene Rohprodukt (19,2 g) wird säulenchromatographisch an 100 g Kieselgel 60 (Teilchengröße: 0,063-0,2 mm) mit Chloroform: Methanol: Aceton im Volumverhältnis von 92:4:4 als Elutionsmittel vorgereinigt. Die den Cyclosporinkomplex enthaltenden Fraktionen (Dünnschichtchromatographie an Platten aus Kieselgel 60 $F_{254}$, DC-Alufolie /Merck/; Entwickler: Hexan und Aceton im Volumverhältnis von 2:1, Anfärbung: Chlor und Tolidin) werden zur Trockne eingedampft. Aus dem Eindampfrückstand (5,28 g) wird das Cyclosporin A säulenchromatographisch abgetrennt. Die Säule enthält 115 g Kieselgel 60 (Teilchengröße: 0,063-0,2 mm). Als Elutionsmittel wird ein Hexan-Aceton-Gradient steigenden Acetongehaltes verwendet. Das Cyclosporin A wird mit 23 Vol.-% Aceton enthaltendem n-Hexan von der Säule gewaschen. Durch Eindampfen der Cyclosporin A enthaltenden Fraktionen erhält man 1,46 g Cyclosporin A.

Beispiel 3

Von einer auf Malzextrakt und Hefeextrakt enthaltendem Schrägagar 5-7 Tage lang gezüchteten Kultur des Stammes Tolypocladium varium sp. nov. CY/93 (NCAIM(P)F 001005) wird mit 5 ml 0,9 gew.-%-iger Kochsalzlösung eine Sporensuspension bereitet. Die Sporensuspension wird zum Beimpfen von 800 ml Nährmedium IC der im Beispiel 1 angegebenen Zusammensetzung verwendet, das sich in einem Erlenmeyerkolben von 3 Liter Volumen befindet und vorher sterilisiert wurde. Der Kolben wird auf dem Schütteltisch (340 $min^{-1}$, Auslenkung 3,5 cm) bei 25 °C 2,5 Tage lang geschüttelt. Mit der erhaltenen Inoculumkultur werden 5 Liter Nährmedium $FC_3$ beimpft. Das Nährmedium befindet sich in einem Laborfermentor von 10 l Volumen und wurde vorher bei 121 °C 45 Minuten lang sterilisiert.

| Zusammensetzung des Nährmediums $FC_3$ | |
|---|---|
| Sorbose | 60 g |
| Trypkasin | 40 g |
| Harnstoff | 2 g |
| Ammoniumsulfat | 12 g |
| Natriumnitrat | 3 g |
| Kaliumdihydrogenphosphat | 2 g |
| Kaliumchlorid | 0,5 g |
| Magnesiumsulfat .7$H_2$O | 0,5 g |
| Mangan(II)sulfat.7$H_2$O | 0,01 g |
| Eisen(II)sulfat .7$H_2$O in 1000 ml Leitungswasser. | 0,01 g |

Der pH-Wert des Nährmediums wird vor dem Sterilisieren auf 5,2 eingestellt.

Die beimpfte Kultur wird auf 25 °C thermostatisiert, gerührt (1000 $min^{-1}$) und mit 300 l/h Luft belüftet. Die Kultur wird 120-168 Stunden lang gegoren, danach enthält die Gärbrühe nach mikrobiologischen Wertmessungen 600 $\mu$g/ml Cyclosporinkomplex. Aus 1 Liter Gärbrühe werden auf die im Beispiel 2 beschriebene Weise 310 mg Cyclosporin A gewonnen.

Beispiel 4

Von einer auf Malzextrakt und Hefeextrakt enthaltendem Schrägagar 5-7 Tage lang gezüchteten Kultur des Stammes Tolypocladium varium sp. nov. CY/93 (NCAIM(P)F 001005) wird mit 5 ml 0,9 gew.-%-iger Kochsalzlösung eine Sporensuspension bereitet. Die Sporensuspension wird zum Beimpfen von 500 ml Nährmedium IC der im Beispiel 1 angegebenen Zusammensetzung verwendet, das sich in einem Erlenmeyerkolben von 3 Liter Volumen befindet und vorher sterilisiert wurde. Der Kolben wird auf dem Schütteltisch (340 $min^{-1}$, Auslenkung 3,5 cm) bei 25 ºC 3 Tage lang geschüttelt. Mit der erhaltenen Inoculumkultur werden 5 Liter Nährmedium $FC_4$ beimpft. Das Nährmedium befindet sich in einem Laborfermentor von 10 Liter Volumen und wurde vorher bei 121 ºC 45 Minuten lang sterilisiert.

| Zusammensetzung des Nährmediums FC$_4$ | |
|---|---|
| Maltose | 80 g |
| Trypkasin | 40 g |
| Harnstoff | 2 g |
| Ammoniumsulfat | 12 g |
| Natriumnitrat | 3 g |
| Kaliumdihydrogenphosphat | 2 g |
| Kaliumchlorid | 0,5 g |
| Magnesiumsulfat .7H$_2$O | 0,5 g |
| Kupfer(II)sulfat . 7 H$_2$O | 0,01 g |
| Mangan(II)sulfat 7 H$_2$O | 0,01 g |
| Eisen(II)sulfat .7H$_2$O in 1000 ml Leitungswasser. | 0,01 g |

Das Nährmedium wird vor dem Sterilisieren auf pH 5,2 eingestellt. Die beimpfte Kultur wird bei 25 °C unter Rühren (1000 min$^{-1}$) und Belüften (300 l/h) 144 Stunden lang gegoren. Am Ende der Gärung enthält die Gärbrühe nach mikrobiologischen Wertmessungen 620$\mu$g/ml Cyclosporinkomplex. Aus 1 Liter Gärbrühe werden auf die im Beispiel 1 beschriebene Weise 305 mg Cyclosporin A gewonnen.

Beispiel 5

Von einer auf Malzextrakt und Hefeextrakt enthaltendem Schrägagar 5-7 Tage lang gezüchteten Kultur des Stammes Tolypocladium varium sp. nov. CY/93 (NCAIM(P)F 001005) wird mit 5 ml 0,9gew.-%-iger Kochsalzlösung eine Sporensuspension bereitet. Die Sporensuspension wird zum Beimpfen von 500 ml Nährmedium IC der im Beispiel 1 angegebenen Zusammensetzung verwendet, das sich in einem Erlenmeyerkolben von 3 Liter Volumen befindet. Der Kolben wird bei 25 °C auf einer Mühlensieb-Schüttelmaschine(340 min$^{-1}$, Auslenkung 3,5 cm) 2 Tage lang geschüttelt. Mit der erhaltenen Inoculumkultur werden 5 Liter Nährmedium FC$_1$ beimpft. Das Nährmedium befindet sich in einem Laborfermentor von 10 Liter Volumen und wurde vorher bei 121 °C 45 Minuten lang sterilisiert. Die beimpfte Kultur wird bei 25 °C unter Rühren (750 min$^{-1}$) und Belüften (300 l/h) gegoren. In der 96. Stunde werden 2 Gew.-% getrennt sterilisierte Maltose zugesetzt, dann wird bis zur 144. Stunde gegoren. Die Gärbrühe enthält zu diesem Zeitpunkt nach mikrobiologischen Wertmessungen 950 $\mu$g/ml Cyclosporinkomplex. Aus den 2,8 Liter Gärbrühe werden auf die im Beispiel 2 beschriebene Weise 2,75 g Cyclosporin A isoliert.

**Patentansprüche**

1. Mikroorganismenstamm Tolypocladium varium sp. nov. CY/93, hinterlegt am 15. Mai 1987 unter der Nummer NCAIM(P)F 001005 in der Nationalen Sammlung Landwirtschaftlicher und Industrieller Mikroorganismen der Budapester Universität für Gartenbau und Lebensmittelindustrie.

2. Verfahren zur Herstellung des Antibioticumkomplexes Cyclosporin und/oder seiner Bestandteile Cyclosporin A, Cyclosporin B und/oder Cyclosporin C durch Züchten eines die genannten Antibiotica biosynthetisierenden Pilzstammes in einem assimilierbare Kohlenstoff- und organische und anorganische Stickstoffquellen sowie Mineralsalze enthaltenden Nährmedium unter aeroben Bedingungen und Abtrennen der gebildeten Produkte, dadurch gekennzeichnet, daß man als die genannten Antibiotica biosynthetisierenden Pilzstamm den Mikroorganismenstamm Tolypocladium varium sp. nov. CY/93, hinterlegt an 15. Mai 1987 unter der Nummer NCAIM(P)F 001005 in der Nationalen Sammlung Landwirtschaftlicher und Industrieller Mikroorganismen der Budapester Universität für Gartenbau und Lebensmittelindustrie, im Nährmedium bei 25 bis 30 °C züchtet sowie gegebenenfalls den erhaltenen Antibioticumkomplex oder seine Bstandteile aus der Gärbrühe isoliert und gegebenenfalls reinigt.

3. Verfahren nach Anspruch 2, dadurch gekennzeichnet, daß man als Stickstoffquelle [ein] Pepton(e), Ammoniumsulfat und/oder Trypkasin und als Kohlenstoffquelle Glucose, Maltose und/oder Sorbit verwendet.

**Claims**

**1.** Microorganism strain *Tolypocladium varium* sp. nov. CY/93, deposited on May 15, 1987 under the number NCAIM (P)F 001005 in the "National Collection of Agricultural and Industrial Microorganisms of the University of Budapest for Horticulture and Food Industry".

**2.** Process for obtaining the complex of antibiotic cyclosporin and/or its components cyclosporin A, cyclosporin B and/or cyclosporin C by culturing a fungi strain biosynthesizing said antibiotics in a nutrient medium containing assimilable carbon sources and organic and inorganic nitrogen sources as well as mineral salts under aerobic conditions and separating the formed products, **characterized by** culturing the microorganism strain, deposited on May 15, 1987 under the number NCAIM (P)F 001005 in the "National Collection of Agricultural and Industrial Microorganisms of the University of Budapest for Horticulture and Food Industry", as said fungi strain biosynthesizing said antibiotics at 25 to 30°C and, optionally, isolating the resulting complex of antibiotic or its components from the fermentation broth and, optionally, purifying it/them.

**3.** Process according to claim 2, **characterized by** using [a] peptone(s), ammonium sulfate and/or trypcasin as nitrogen source and glucose, maltose and/or sorbitol as carbon source.

**Revendications**

**1.** Souche de micro-organismes tolypocladiam varium sp. nov. CY/93, déposée le 15 mai 1987 sous le numéro NCAIM(P)F 001005 dans la collection nationale de micro-organismes agricoles et industriels de l'université de Budapest pour l'horticulture et l'industrie des denrées alimentaires.

**2.** Procédé pour obtenir des complexes de l'antibiotique cyclosporine et/ou ses composants cyclosporine A, cyclosporine B et/ou cyclosporine C par la culture d'une souche de champignon biosynthétisant lesdits antibiotiques dans un milieu de culture contenant des sources de carbone et d'azote organique et anorganique assimitables ainsi que des sels minéraux dans des conditions aérobies et de séparation des produits formés, caractérisé en ce qu'on cultive en tant que souche de champignon biosynthétisant les antibiotiques la souche de micro-organismes tolypocladiam varium sp. nov. CY/93, déposée le 15 mai 1987 sous le numéro NCAIM(P)F 001005 à la collection nationale de micro-organismes agricoles et industriels de l'université de Budapest pour l'horticulture et l'industrie des denrées alimentaires dans un milieu de culture à une température entre 25°C et 30°C et que l'on isole le cas échéant du bouillon de fermentation le complexe d'antibiotiques ou ses composants obtenus et qu'on les purifie le cas échéant.

**3.** Procédé selon la revendication 2, caractérisé en ce que l'on utilise comme source d'azote un [des] peptone(s), du sulfate d'ammonium et/ou de la trypcasine et, en tant que source de carbone, du glucose, maltose et/ou du sorbitol.

12